(19) **Europäisches Patentamt**

**European Patent Office**

**Office européen des brevets**

(11) **EP 0 774 443 B1**

(12) **EUROPÄISCHE PATENTSCHRIFT**

(45) Veröffentlichungstag und Bekanntmachung des Hinweises auf die Patenterteilung:
**19.05.1999 Patentblatt 1999/20**

(51) Int. Cl.[6]: **C01G 23/053**, B01J 21/00, A61K 7/42, A61K 7/00, C09C 1/36

(21) Anmeldenummer: **96117831.6**

(22) Anmeldetag: **07.11.1996**

(54) **Nanodisperses Titandioxid, Verfahren zu dessen Herstellung und seine Verwendung**

Nanodisperse titanium dioxide, process for its preparation and its use

Dioxyde de titane nanodisperse, procédé pour sa fabrication et son utilisation

(84) Benannte Vertragsstaaten:
**DE ES FI FR GB IT NL**

(30) Priorität: **20.11.1995 DE 19543204**

(43) Veröffentlichungstag der Anmeldung:
**21.05.1997 Patentblatt 1997/21**

(73) Patentinhaber: **BAYER AG**
**51368 Leverkusen (DE)**

(72) Erfinder:
 • **Wiederhöft, Gerhard**
 **47802 Krefeld (DE)**
 • **Bütje, Kai, Dr.**
 **47229 Duisburg (DE)**
 • **Barenthien, Peter-Joachim**
 **47239 Duisburg (DE)**
 • **Bödiger, Michael, Dr.**
 **41539 Dormagen (DE)**
 • **Alberts, Heinrich, Dr.**
 **51519 Odenthal (DE)**

(56) Entgegenhaltungen:
**FR-A- 2 677 012        US-A- 5 028 417**

 • **PATENT ABSTRACTS OF JAPAN vol. 014, no. 470 (C-0769), 15.Oktober 1990 & JP 02 194063 A (TEIKA CORP), 31.Juli 1990,**
 • **PATENT ABSTRACTS OF JAPAN vol. 012, no. 110 (C-486), 8.April 1988 & JP 62 235215 A (NIPPON SHOKUBAI KAGAKU KOGYO CO LTD), 15.Oktober 1987,**
 • **DATABASE WPI Section Ch, Week 8510 Derwent Publications Ltd., London, GB; Class A35, AN 85-059891 XP002026350 & JP 60 017 190 A (TORAY IND INC) , 29.Januar 1985**

**Beschreibung**

Die vorliegende Erfindung betrifft nanodisperses Titandioxid, ein Verfahren zu dessen Herstellung sowie seine Verwendung.

Unter nanodispersem Titandioxid ("Nano-TiO$_2$") im Sinne der vorliegenden Erfindung werden Rutile, Anatase und amorphes Titandioxid mit einer Teilchengröße von 1 bis 100 nm, bevorzugt 1 bis 10 nm verstanden bzw. in Dispersion vorliegendes Titandioxid mit vorgenannter Teilchengröße. Für solche Titandioxidpartikel beginnt sich eine Reihe interessanter technischer Anwendungen abzuzeichnen:

- als UV-Schutzkomponente in Kosmetika, Kunststoffen, Silikonharzen und Lacken, wobei die durch die geringe Teilchengröße bedingte Transparenz eine besonders wünschenswerte Eigenschaft der Partikel ist;

- als Flammschutzmittel und zur Erhöhung des Brechungsindex von Silikonen und Kunststoffen, wie in FR 2 682 369 beschrieben;

- im Umweltschutz zur Zerstörung organischer, auch halogenhaltiger Schadstoffe in Abwässern durch Photokatalyse;

- zur Beschleunigung des Zerfalls (bio-)abbaubarer Polymerer;

- als Trägermaterial für neuartige Farbstoff-Solarzellen, wie sie z.B. in PCT-WO 93/20569 beschrieben sind;

- gemeinsam mit auf dem gleichen Weg hergestelltem SiO$_2$ als Komponente in speziellen Gläsern.

Die Anwendung dieser TiO$_2$-Nanopartikel wird jedoch zur Zeit noch dadurch eingeschränkt, daß bisher kein wirtschaftliches Verfahren bekannt ist, das Nano-TiO$_2$ der genannten Teilchengröße im industriellen Maßstab zu liefern imstande ist.

Die wichtigsten Methoden zur Synthese von Nanoteilchen lassen sich unter dem Oberbegriff Sol-Gel-Verfahren zusammenfassen. Sie sind in einer großen Anzahl von Zeitschriftenartikeln und Patenten beschrieben.

Unter dem Sol-Gel-Verfahren im engeren Sinne versteht man die Alkoxidmethode, d.h. die sorgfältig gesteuerte, häufig basen- oder säurekatalysierte Hydrolyse von Metall-Alkoxiden und ähnlichen molekularen Vorläufern in Mischungen aus Wasser und einem oder mehreren organischen Lösungsmitteln. In der Regel wird als Lösungsmittel derselbe Alkohol verwendet, der dem Alkoxid zugrundeliegt. Nachteil dieser Verfahren ist, daß teure Edukte und eine aufwendige Verfahrenstechnik erforderlich sind. Zudem weisen die Produkte einen hohen Kohlenstoffgehalt auf.

Ursprünglich an Siliciumverbindungen entwickelt, wird das Sol-Gel-Verfahren zunehmend auch zur Synthese von Nano-Titandioxid gemäß der Gleichung

$$Ti(OR)_4 + 2\,H_2O \rightarrow TiO_2 + 4\,ROH$$

eingesetzt (siehe z.B. J. Livage. Mat. Sci. Forum 152-153 (1994), 43-54; J.L. Look und C.F. Zukoski, J. Am. Ceram. Soc. 75 (1992), 1587-1595; WO 93/05875).

Durch geeignete Wahl der Reaktionsbedingungen gelingt häufig die Synthese monodisperser Partikel, d.h. solcher mit einer sehr engen Teilchengrößenverteilung, wobei der Durchmesser der Partikel im Bereich von Mikrometern bis herab zu wenigen Nanometern liegt. Ein Beispiel dieser speziellen Reaktionsführung ist das Arbeiten in Mikroemulsionen` womit es gelingt, die Teilchengröße zu begrenzen (siehe z.B. D. Papoutsi et al., Langmuir 10 (1994), 1684-1689).

Edukte für alle Sol-Gel-Reaktionen zur Herstellung von Nano-TiO$_2$, ob konventionell oder in Mikroemulsion, sind Titanalkoholate Ti(OR)$_4$, deren Alkylreste R üblicherweise 2 bis 4 Kohlenstoffatome enthalten. Aufgrund des hohen Preises dieser Alkoholate und des speziellen Handlings (Schutzgas, strenger Feuchtigkeitsausschluß zur Vermeidung vorzeitiger Hydrolyse) kommen die genannten Umsetzungen jedoch für einen großtechnischen Prozeß nicht in Betracht.

Das Arbeiten in Mikroemulsion hat zudem den Nachteil, daß die Raum-Zeit-Ausbeute infolge der häufig niedrigen Konzentrationen der Reaktionspartner gering ist und daß große Mengen von Wasser/Lösungsmittel/Tensid-Mischungen anfallen, die entsorgt werden müssen.

Kürzlich ist ein alternativer, nicht-hydrolytischer Sol-Gel-Prozeß vorgeschlagen worden (S. Acosta et al., Better Ceramics through Chemistry VI (1994), 43-54), der die Umsetzung von Metallhalogeniden mit Sauerstoffdonatoren wie Ethern oder Alkoxiden vorsieht.

Eine weitere Gruppe von Methoden zur Herstellung ultrafeiner Titandioxid-Partikel sind die sogenannten CVR (Chemical Vapor Reaction)-Verfahren, die auf einer Umsetzung verdampfbarer Metallverbindungen (in der Regel Alkoxide) mit Sauerstoff (Luft) oder Wasserdampf in der Gasphase beruhen, wie z.B. in US 4 842 832 und EP-A 214 308 beschrieben. Nach solchen Verfahren hergestellte Pulver sind zwar bereits in Kleinmengen auf dem Markt erhältlich, aber extrem teuer.

Von den bisher bekannt gewordenen großtechnisch beherrschten Verfahren zur Herstellung von feinteiligem (subpigmentärem) Titandioxid, nämlich Hydrolyse von $TiCl_4$ (GB-A 22 05 288), Herstellung von Rutilkeimen im Rahmen des Sulfatprozesses (EP-A 444 798, EP-A 499 863) und Peptisation von sulfatfrei gewaschenem Titandioxidhydrat mit einbasigen Säuren (EP-A 261 560, US 2 448 683), liefert keines ein Produkt, das in seiner Feinteiligkeit und Transparenz mit Sol-Gel-Materialien vergleichbar wäre.

Aus der Literatur ist weiterhin bekannt, die Hydrolyse von $TiCl_4$ unter hydrothermalen Bedingungen durchzuführen, wobei je nach den Reaktionsbedingungen (Konzentration, Temperatur, pH-Wert, Mineralisatoren) Nano-Anatase und - Rutile erhalten werden (H. Cheng et al., Chem. Mater. 7 (1995), 663-671). Aufgrund der aufwendigen Verfahrenstechnik ist jedoch zu bezweifeln, daß mit dieser Methode ein marktfähiges Produkt erhalten werden kann.

Relevanter Stand der Technik ist ferner die FR-A-2 667 012.

Aufgabe war es daher, ein nanodisperses Titandioxid zur Verfügung zu stellen, das es gestattet, transparente Sole daraus herzustellen, sowie ein Verfahren zu dessen Herstellung. Das Verfahren zur Herstellung von Nano-Titandioxid sollte die Wirtschaftlichkeit und relativ einfache Verfahrenstechnik eines großindustriellen Prozesses aufweisen, und das Verfahrensprodukt sollte die günstigen Eigenschaften (Feinteiligkeit und Transparenz) eines Sol-Gel-Produktes haben.

Gegenstand der Erfindung ist ein nanodisperses Titandioxid mit einem Maximum der mittels einer Ultrazentrifuge bestimmten Teilchengrößenverteilung zwischen 1 und 10 nm, mit weniger als 0,1 Gew.-% Kohlenstoff in Form organischer Verbindungen oder Reste und mit einer Transparenz von mindestens 99 %, gemessen in 5 gew.-%iger wäßrig-salzsaurer Lösung zwischen 400 und 700 nm in 180°/d-Geometrie in einer Schichtdicke von 10 µm.

Das erfindungsgemäße Titandioxid kann auch mit 0,1 bis 50 Gew.-%, vorzugsweise 5 bis 30 Gew.-%, bezogen auf $TiO_2$, eines oder mehrerer Oxide, Hydroxide oder wasserhaltiger Oxide eines oder mehrerer Elemente aus der Gruppe bestehend aus Aluminium, Silizium, Zirkonium, Zinn, Magnesium, Zink, Cer und Phosphor beschichtet sein.

Ein weiterer Gegenstand dieser Erfindung ist ein transparentes Titandioxid-Sol, das 1 bis 20 Gew.-% des erfindungsgemäßen nanodispersen Titandioxids in einem oder mehreren Lösungsmitteln aus der Gruppe bestehend aus Wasser und Alkoholen, die 1 bis 10 Kohlenstoffatome und eine oder mehrere Hydroxidgruppen im Molekül aufweisen, enthält.

Gegenstand der Erfindung ist weiterhin ein Verfahren zur Herstellung des erfindungsgemäßen nanodispersen Titandioxids, welches dadurch gekennzeichnet ist, daß

a) zu einer alkalisch reagierenden Flüssigkeit bei erhöhter Temperatur eine schwefelsaure Titanylsulfatlösung zugegeben wird, bis die so erhaltene Mischung sauer reagiert, also Schwefelsäure im Überschuß vorliegt, oder eine alkalisch reagierende Flüssigkeit und eine schwefelsaure Titanylsulfatlösung bei erhöhter Temperatur simultan unter guter Vermischung in einem Behälter so zusammengeführt werden, daß die erhaltene Mischung sauer reagiert, also Schwefelsäure im Überschuß vorliegt,

b) die unter a) erhaltene Mischung abgekühlt wird,

c) anschließend eine einbasige Säure zu der nach b) erhaltenen Mischung zugegeben wird, wobei die unter a) gebildeten Titandioxidnanopartikel ausflocken,

d) der unter c) gebildete Flockungsniederschlag abfiltriert und mit der gleichen einbasigen Säure wie unter c) gewaschen wird.

Um das erfindungsgemäße transparente Titandioxid-Sol zu erhalten, wird der nach Reaktionsschritt d) erhaltene Niederschlag in Wasser und/oder Alkoholen, die 1 bis 10 Kohlenstoffatome und eine oder mehrere Hydroxidgruppen im Molekül aufweisen, aufgenommen.

Überraschenderweise gelingt die Herstellung des erfindungsgemäßen nanodispersen $TiO_2$ auch im Rahmen eines großtechnischen Prozesses, nämlich der $TiO_2$-Pigmentherstellung nach dem Sulfatverfahren, und ist damit sehr einfach und wirtschaftlich.

Der nach dem erfindungsgemäßen Verfahren erhaltene Filterrückstand (nach Schritt d)) kann anorganisch und/oder organisch nachbehandelt werden.

Als Edukt (schwefelsaure Titanylsulfatlösung) ist im Prinzip jede Schwefelsäure im Überschuß enthaltende Titanylsulfatlösung geeignet. Verunreinigungen von Metallen, die lösliche Sulfate und Chloride bilden, wie z.B. Eisen, Magnesium, Aluminium und Alkalimetalle stören beim Herstellungsprozeß im Prinzip nicht, es sei denn, die genannten Elemente wirken sich für den jeweiligen Anwendungszweck schon in Spuren nachteilig aus. Damit ist die Durchführung des erfindungsgemäßen Verfahrens im großtechnischen Maßstab möglich. Als Edukt kann z.B. Schwarzlösung eingesetzt werden, wie sie im Sulfatprozeß nach dem Aufschluß von Ilmenit und/oder Titanschlacke mit Schwefelsäure, Lösen des erhaltenen Aufschlußkuchens in Wasser und Klärfiltration anfällt.

Das erfindungsgemäße Herstellungsverfahren ist jedoch nicht auf Schwarzlösung als Edukt beschränkt. Andere Verfahren zur Herstellung einer als Edukt geeigneten schwefelsauren Titanylsulfatlösung sind beispielsweise:

a) Auflösen/Aufschließen von Titandioxid und $TiO_2$-Hydraten, z.B. Orthotitansäure, Metatitansäure, in überschüssiger $H_2SO_4$;

b) Auflösen/Aufschließen von Alkali- und Magnesiumtitanaten, auch in wasserhaltiger Form, in überschüssiger $H_2SO_4$;

c) Umsetzung von $TiCl_4$ mit überschüssiger $H_2SO_4$ unter Bildung von $TiOSO_4$ und HCl, wie in DE-A 42 16 122 beschrieben.

Die Produkte, insbesondere aus a) und aus c) kommen als schwefelsaure Titanylsulfatlösungen bevorzugt dann zum Einsatz, wenn Spuren von Fremdmetallen (z.B. Eisen) im erfindungsgemäßen Produkt unerwünscht sind.

Für eine wirtschaftlich sinnvolle Arbeitsweise enthalten die erfindungsgemäß einzusetzenden schwefelsauren Titanylsulfatlösungen bevorzugt 100 bis 260, besonders bevorzugt 170 bis 230 g Titan/l, berechnet als $TiO_2$. Der Säureüberschuß beträgt bevorzugt 0,3 bis 4,0, besonders bevorzugt 0,5 bis 1,5 mol $H_2SO_4$ pro mol $TiOSO_4$.

Als alkalisch reagierende Flüssigkeiten werden vorzugsweise wäßrige Lösungen von Natrium-, Kaliumhydroxid oder Ammoniak eingesetzt; prinzipiell können auch Carbonate von Natrium, Kalium und Ammonium eingesetzt werden, die jedoch aufgrund der starken $CO_2$-Entwicklung weniger geeignet sind. Besonders bevorzugt ist Natronlauge, anhand derer die Verfahrensdurchführung näher erläutert wird.

Die Natriumhydroxidmenge ist beispielsweise so zu bemessen, daß das Natriumhydroxid im stöchiometrischen Unterschuß z. B. bezüglich der "freien Schwefelsäure" nach Reaktionsschritt a) vorliegt Unter "freier Schwefelsäure" im Rahmen des Sulfatprozesses der $TiO_2$-Produktion versteht der Fachmann den Gesamtschwefelsäuregehalt abzüglich des in Form von Fremdmetallsulfaten (in erster Linie $FeSO_4$) gebundenen Anteils, d.h. die Summe aus $H_2SO_4$ und als $TiOSO_4$ gebundener Schwefelsäure; der letztgenannte Anteil liegt nach der Hydrolyse als $H_2SO_4$ vor.

Die Natronlaugemenge wird im stöchiometrischen Unterschuß bezüglich der beiden Reaktionen

$$H_2SO_4 + 2\ NaOH \rightarrow Na_2SO_4 + 2\ H_2O$$

$$TiOSO_4 + 2\ NaOH \rightarrow TiO_2 + Na_2SO_4 + H_2O$$

eingestellt, wobei der Unterschuß vorzugsweise so gewählt wird, daß der pH-Wert am Ende des Reaktionsschrittes a) vorzugsweise kleiner als 2 ist.

Bevorzugt wird als Natronlauge eine wäßrige Natriumhydroxidlösung verwendet, deren Konzentration etwa 5 bis 10 Gew.-% NaOH beträgt.

Die Umsetzung der unterschüssigen Natronlauge mit der schwefelsauren Titanylsulfatlösung erfolgt bevorzugt dergestalt, daß die auf etwa 60 bis 100°C erwärmte Natronlauge vorgelegt wird und man in diese Lösung die schwefelsaure Titanylsulfatlösung einlaufen läßt.

Vorzugsweise kann die Umsetzung in Schritt a) auch so durchgeführt werden, daß die beiden Reaktionspartner gleichzeitig unter Rühren bei Temperaturen zwischen 60 und 100°C zusammengegeben werden.

Der Reaktionsschritt a) sollte bevorzugt unter intensivem Rühren und bei Temperaturen von 60 bis 100°C durchgeführt werden.

Der alkalische pH-Bereich in der Vorlage sollte möglichst rasch (in bevorzugt weniger als 5 Minuten) durchlaufen und verlassen werden.

Die Mischung sollte vorzugsweise nach dem Reaktionsschritt a) auf Temperaturen unterhalb 60°C abgeschreckt werden und dann gegebenenfalls 1/2 bis 2 Stunden bei dieser Temperatur unter Rühren gehalten werden.

Die so erhaltene Mischung ist mehr oder weniger getrübt (trübes Sol). Derartige Mischungen werden als sogenannte Hydrolysekeime im $TiO_2$-Sulfatprozeß eingesetzt. Als transparente Sole sind sie ungeeignet.

Nach der Abkühlung wird mittels einer einbasigen Säure ausgeflockt und durch Filtration der Flockungsniederschlag isoliert. Dabei handelt es sich um nanodisperses Titandioxid mit einer Teilchengröße zwischen 1 und 10 nm, mit weniger als 0,1 Gew.-% Kohlenstoff und einer Transparenz von mindestens 99 % (siehe oben).

Bevorzugt wird vor der Zugabe der einbasigen Säure geklärt. Dies kann in einfacher Weise durch Sedimentation, d.h. mindestens 12stündiges ruhiges Stehen und Dekantieren erfolgen. Es ist aber auch möglich, zu zentrifugieren

4

oder zu filtrieren, falls erforderlich mit einer Filterhilfe.

Durch die Zugabe der einbasigen Mineralsäure werden die in Reaktionsschritt a) gebildeten Nanopartikel reversibel ausgeflockt. Die erhaltenen voluminösen Flocken sind aufgrund ihrer Größe (bevorzugt 1 bis 10 µm) gut zentrifugierbar und filtrierbar. Die bevorzugte einbasige Säure ist Salzsäure, anhand derer die weitere Verfahrensweise näher erläutert werden soll. Bei Einsatz anderer einbasiger Mineralsäuren ist entsprechend zu verfahren.

Bevorzugt sollte die HCl-Konzentration in der Salzsäure nicht kleiner als 1 molar sein; bevorzugt wird sie auf 1 bis 6 molar, besonders bevorzugt auf 1 bis 4 molar eingestellt.

Als Filtertücher werden solche aus säurefestem Material (beispielsweise Polypropylen) bevorzugt. Besonders geeignet sind die dem Fachmann bekannten säurefesten Filtertücher, die im Rahmen des Sulfatprozesses auch zur Isolierung von $TiO_2$-Hydrolysat verwendet werden, sowie Membranfilter auf der Basis von Cellulose, Celluloseethern oder -estern.

Der Niederschlag wird anschließend ausgewaschen, bevorzugt mit der gleichen einbasigen Säure, die auch zur Flockung verwendet wird. Im Fall von Salzsäure ist 3 bis 6 molare Salzsäure als Waschflüssigkeit besonders geeignet.

Die erhaltenen (salz-)sauren Niederschläge (Pasten) enthalten je nach Filteraggregat und Ausgangsmaterial 20 bis 40, typischerweise etwa 30 Gew.-% $TiO_2$, Rest Waschsäure und gegebenenfalls geringe Verunreinigungen.

Die Niederschläge liefern, in Wasser aufgenommen, "Lösungen" (Sole), die bis auf eine leichte Opaleszenz (Tyndall-Effekt) klar, transparent und farblos bzw. fast farblos sind. In diesen Solen liegt das $TiO_2$ vollständig als Nanopartikel vor, dessen Durchmesser zwischen 1 und 10 nm liegt.

Es gelingt auf diese Weise, stark saure, nahezu völlig transparente (wasserklare) Sole mit bis zu ca. 20 Gew.-% $TiO_2$ herzustellen. Die Transparenz der Sole liegt bei einer Konzentration von 5 Gew.-% $TiO_2$ über den gesamten sichtbaren Spektralbereich über 99 % (gemessen in 180°/d-Geometrie).

Durch Zugabe einbasiger Mineralsäuren, z.B. HCl, können die Nanopartikel wieder ausgeflockt, abfiltriert und gewaschen werden. Sie sind in dieser Form bei Aufbewahrung um 0°C wochenlang ohne Veränderung haltbar.

Ähnliche Sole sind auch in polaren organischen Lösungsmitteln, in erster Linie in ein- und mehrwertigen kurzkettigen Alkoholen, wie z.B. Ethanol und Butandiol(1,4) herstellbar. Die Alkohole weisen bevorzugt 1 bis 10 Kohlenstoffatome im Molekül auf.

Durch Abdampfen der Flüssigkeit und der anhaftenden Säure bei möglichst niedriger Temperatur im Vakuum oder über NaOH (Raumtemperatur, Gefriertrocknung) erhält man aus den Pasten glasartige Xerogele, die bei nicht zu weitgehender Abtrennung von $H_2O$ und HCl in Wasser klar dispergierbar sind.

Durch Dialyse gegen verdünnte einbasige Mineralsäuren gelingt es, gegebenenfalls vorhandene Schwermetallionen abzureichern.

Bei der Dialyse des salzsauren Sols gegen destilliertes Wasser führt die Kondensation der Nanoteilchen zur Bildung von Gelen, die bei ausreichend hoher $TiO_2$-Konzentration monolithisch sein können.

Bei Anwendungen, in denen Säureüberschüsse störend wirken, können die erfindungsgemäßen Teilchen anschließend in prinzipiell bekannter Weise im neutralen pH-Bereich stabilisiert werden, z.B. mit Acetylaceton (WO 93/05875) oder mit Hydroxycarbonsäuren (EP-A 518 175).

Das nanodisperse Titandioxid wird als UV-Schutzkomponente in Kosmetika, Kunststoffen, Silikonharzen und Lacken verwendet.

Sofern eine Herabsetzung der Photoaktivität gewünscht wird, können die Nanoteilchen anorganisch beschichtet (nachbehandelt) werden, wobei die Beschichtung wie bei pigmentärem $TiO_2$ mit Oxiden, Hydroxiden oder wasserhaltigen Oxiden eines oder mehrerer der folgenden Elemente erfolgt: Al, Si, Zr, Sn, Mg, Zn, Ce, P. Die anzuwendenden Mengen betragen 0,1 bis 50, bevorzugt 5 bis 30 Gew.-% bezogen auf $TiO_2$.

Die anorganische Nachbehandlung ist nicht erforderlich, vielmehr unerwünscht, wenn das Produkt als Katalysator zum photochemischen Abbau organischer Verbindungen (Polymere, Schadstoffe) oder als Träger für Farbstoff-Solarzellen eingesetzt wird.

Die Erfindung soll anhand der nachfolgenden Beispiele näher erläutert werden.

## Beispiele

**Beispiel 1** (Herstellung des Nano-$TiO_2$ aus sogenannter Schwarzlösung)

In einem doppelwandigen, beheizbaren 6 l-Planschlifftopf mit mechanischem Rührer, Thermometer, Rückflußkühler und mit Bodenventil zum Ablassen des Produktes werden 1400 ml 7,5 gew.-%ige Natronlauge auf 85°C erwärmt. In einem 1 l-Dreihalskolben mit Rührer, Rückflußkühler, Heizpilz und Bodenablaßventil werden 804 ml einer nach dem Sulfatverfahren hergestellten $FeSO_4$-haltigen Schwarzlösung ($d_{60°C}$ = 1,566 g/ml; 13,83 Gew.-% $TiO_2$ entspricht 217 g/l; 28,39 Gew.-% freie $H_2SO_4$) auf 60°C erwärmt. Unter intensivem Rühren läßt man die Schwarzlösung innerhalb von gut 3 Minuten aus einer Glasdüse in die vorgelegte Natronlauge einlaufen, wobei sich vorübergehend ein dichter, dunkler Niederschlag bildet. Durch die Neutralisationswärme erhöht sich die Temperatur der Mischung auf 92°C. Nach etwa 5 minütigem Nachrühren ist die Mischung nur noch leicht getrübt. Anschließend wird sie in 20 Minuten unter weiterem Rühren auf 30°C abgekühlt.

Zu 244 ml einer so hergestellten Mischung werden innerhalb von 5 Minuten bei Raumtemperatur 244 ml halbkonzentrierter Salzsäure (20,6 Gew.-%, ca. 6,2 molar) getropft. Es bildet sich ein weißer Niederschlag. Nach 1stündigem Stehen zur Vervollständigung der Fällung wird der Niederschlag über ein Cellulosenitratfilter abgesaugt und portionsweise mit insgesamt 900 ml der obengenannten Salzsäure gewaschen.

Es werden 32,5 g einer weißen Paste mit 34,5 Gew.-% $TiO_2$ (entspricht 58 % der Theorie), 14,7 Gew.-% HCl, 2,7 Gew.-% $SO_4^{2-}$ und 170 ppm Fe erhalten.

10,8 g der Paste werden in 32,1 g destilliertem Wasser gelöst. Die "Lösung" enthält ca. 8,3 Gew.-% $TiO_2$ und 3,6 Gew.-% HCl und ist nahezu klar.

**Beispiel 2** (Herstellung des Nano-$TiO_2$ aus $TiOSO_4$-Lösung)

Es wurde wie in Beispiel 1 gearbeitet. Als Titan-Edukt wird anstelle der Schwarzlösung jedoch eine durch Auflösen von Natriumtitanat in Schwefelsäure erhaltene Titanylsulfatlösung (804 ml; d = 1,272 g/ml; 8,2 Gew.-% $TiO_2$, 23,5 Gew.-% $H_2SO_4$) verwendet. Die Vorlage enthält wie in Beispiel 1 1400 ml 7,5 gew.-%ige NaOH, die Zulaufzeit der Titanylsulfatlösung beträgt 4 Minuten. Nach dem Abkühlen auf Raumtemperatur resultiert eine weiße Suspension.

400 ml dieser Suspension werden innerhalb von 10 Minuten mit 400 ml halbkonzentrierter Salzsäure versetzt. Nach 1stündigem Stehen zur Vervollständigung der Fällung wird der Niederschlag über ein Cellulosenitratfilter abgesaugt und portionsweise mit insgesamt 1700 ml der genannten Salzsäure gewaschen.

Der Filterkuchen wird weitere 22 h bei Raumtemperatur trockengesaugt, wobei ein gelbliches, glasartiges Xerogel (15,6 g) mit 59,8 Gew.-% $TiO_2$ (entspr. 65 % der Theorie), 9,8 Gew.-% HCl und 1,23 % Sulfat resultiert.

5 g des Xerogels werden in 20 g destilliertem Wasser gelöst. Man erhält ein bis auf leichte Opaleszenz transparentes Sol mit ca. 11,8 Gew.-% $TiO_2$ und 1,94 Gew.-% HCl.

**Beispiel 3** (Herstellung eines $TiO_2$-Gels durch Dialyse)

41,5 g einer gemäß Beispiel 1 erhaltenen Paste werden mit 46 g obengenannter Salzsäure zu einer Suspension mit 10,8 Gew.-% $TiO_2$, 18,1 Gew.-% HCl und 0,92 Gew.-% $SO_4^{2-}$ vermischt. 34,2 g dieser Mischung werden in einem Cellophanschlauch 3,5 Stunden gegen destilliertes Wasser dialysiert, wobei aus der anfangs trüben Suspension ein transparentes Sol wird. Das Sol (39,9 g) enthält nach der Dialyse 8,9 Gew.-% $TiO_2$, 0,85 Gew.-% HCl und 0,89 Gew.-% $SO_4^{2-}$.

Nach 24stündiger Lagerung bei Raumtemperatur ist ein starker Viskositätsanstieg zu verzeichnen, nach insgesamt 48stündiger Lagerung hat sich das Sol in ein nicht mehr fließfähiges, monolithisches, transparentes, schneidbares Gel umgewandelt.

**Vergleichsbeispiel 1** (Herstellung eines $TiO_2$-Sols nach dem Stand der Technik: US 2 448 683)

620 g eines gereinigten Titandioxidhydratschlamms aus dem Sulfatprozeß mit ca. 19,5 Gew.-% $TiO_2$ (121 g) und ca. 7 Gew.-% $H_2SO_4$ bezogen auf $TiO_2$ werden mit 50 gew.-%iger Natronlauge auf pH 7,1 bei Raumtemperatur neutralisiert. Nach dem Filtrieren wird der Filterkuchen mit ca. 3 l destilliertem Wasser gewaschen, bis der $BaSO_4$-Test im Filtrat negativ ist. 400 g des Filterkuchens mit ca. 28 Gew.-% $TiO_2$ und ca. 0,3 Gew.-% $SO_4^{2-}$ bezogen auf $TiO_2$ werden mit 5 Gew.-% HCl (bezogen auf $TiO_2$) in Form konzentrierter Salzsäure sowie mit so viel Wasser versetzt, daß die Mischung 25 Gew.-% $TiO_2$ enthält. Nach 30minütigem Rühren bei Raumtemperatur resultiert ein dünnflüssiges weißes Sol.

Beim Verdünnen mit Wasser auf 5 bis 10 Gew.-% $TiO_2$ bleibt das Sol im Gegensatz zu den nach Beispiel 1 und 2 erhaltenen nahezu wasserklaren Solen weiß und milchig. Auch durch Erhöhung der HCl-Konzentration wird es nicht transparent.

Transmissionsmessungen

Eine Probe der nach Beispiel 1 erhaltenen Paste wird mit halbkonzentrierter Salzsäure und destilliertem Wasser auf eine Konzentration von 5 Gew.-% $TiO_2$ und 2,5 Gew.-% HCl eingestellt. Das nach Vergleichsbeispiel 1 erhaltene Sol wird mit destilliertem Wasser auf 5 Gew.-% $TiO_2$ verdünnt. (Wegen Flockungserscheinungen ist es nicht möglich, die HCl-Konzentration auf 2,5 Gew.-% einzustellen).

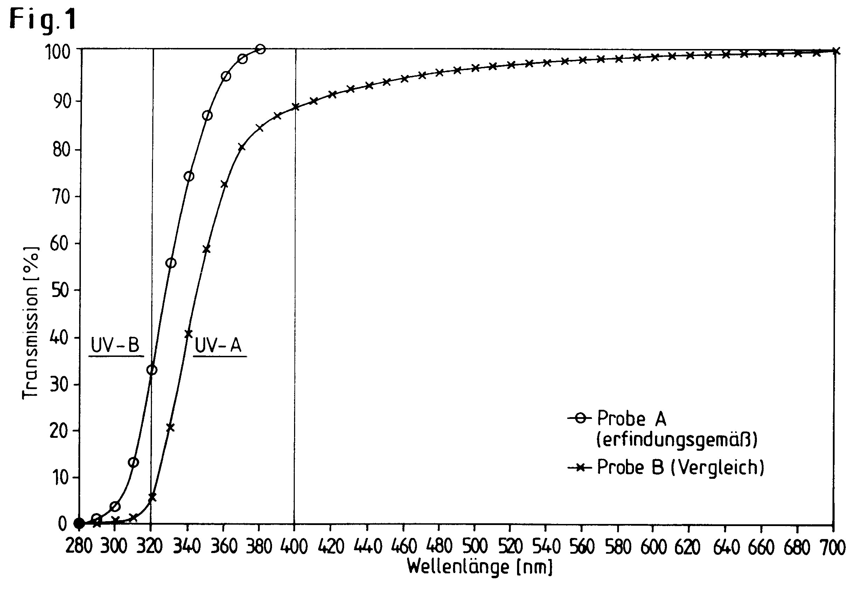

Die UV/VIS-Spektren werden in einer Schichtdicke von 10µm in 180°/d-Geometrie gemessen und sind in Fig. 1 wiedergegeben.

Die Abbildung zeigt, daß beide Proben eine hohe Absorption im ultravioletten Spektralbereich aufweisen, das erfindungsgemäße Nano-TiO$_2$ (Probe A) im sichtbaren Spektralbereich (400 bis 700 nm) aber wesentlich transparenter ist als das Sol nach dem Stand der Technik (Probe B). Weiterhin ist der Wendepunkt der steil verlaufenden Absorptionskante bei dem erfindungsgemäßen Sol gegenüber dem Vergleichssol kurzwellig verschoben.

Teilchenerößenmessungen

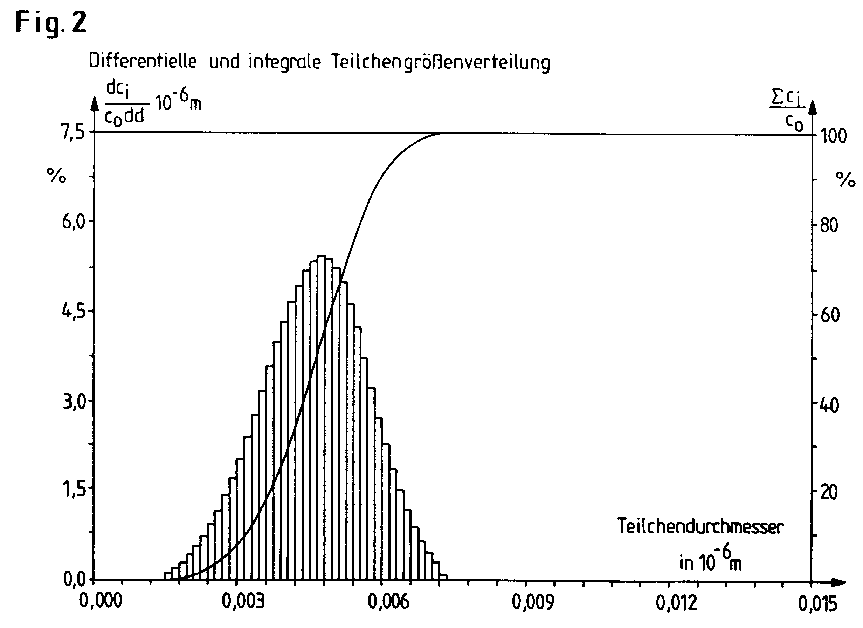

Die Teilchengrößenverteilung einer wie in Beispiel 1 erhaltenen Pastenprobe wird mittels Ultrazentrifuge ermittelt (Lösungsmittel: Wasser/HCl). Der mittlere Durchmesser liegt danach bei ca. 4,5 nm. Die Verteilung ist in Fig. 2 dargestellt.

Für das Vergleichssol aus Vergleichsbeispiel 1 wird mit demselben Gerät ein mittlerer Durchmesser von 36 nm bestimmt.

Bewitterungstests an nicht nachbehandeltem Nano-TiO$_2$ in ABS

Zwei Proben eines Acrylnitril-Butadien-Styrol-Copolymeren, von denen eine mit 5 Gew.-% des erfindungsgemäßen Nano-TiO$_2$ und eine unbehandelt ist, werden in einem Xenon-Weatherometer mit einer Bestrahlungsstärke von 0,35 W/m$^2$ bewittert. Der Sprühzyklus beträgt 102 min. (trocken): 18 min. (Besprühung mit destilliertem Wasser). Der Glanz unter einem Winkel von 60° wird in Abhängigkeit von der Bewitterungsdauer mit einem Farbmeßgerät Dataflash 2000 gemessen (ASTM D 1925). Der Polymerabbau an der Probenoberfläche äußert sich in dieser Versuchsanordnung in einem Glanzabfall. Die Ergebnisse sind in der folgenden Tabelle zusammengefaßt.

| Bewitterungszeit (Stunden) | ABS mit 5 % Nano-TiO$_2$ Glanz | ABS ohne TiO$_2$ Glanz |
|---|---|---|
| 0 | 86 | 92 |
| 250 | 4 | 88 |
| 500 | 3 | 86 |
| 1000 | 3 | 52 |

Die Werte aus der Tabelle zeigen, daß das nanodisperse Titandioxid den Glanzabfall und damit den Abbau des Polymeren oder allgemein ausgedrückt von organischen Materialien sehr stark beschleunigt.

**Patentansprüche**

1.  Nanodisperses Titandioxid mit einem Maximum der mittels einer Ultrazentrifuge bestimmten Teilchengrößenverteilung zwischen 1 und 10 nm, mit weniger als 0,1 Gew.-% Kohlenstoff in Form organischer Verbindungen oder Reste und mit einer Transparenz von mindestens 99 %, gemessen in 5 gew.-%iger wäßrig-salzsaurer Lösung zwischen 400 und 700 nm in 180° /d-Geometrie in einer Schichtdicke von 10 μm.

2.  Nanodisperses Titandioxid gemäß Anspruch 1, dadurch gekennzeichnet, daß das Titandioxid mit 0,1 bis 50 Gew.-%, bezogen auf TiO$_2$, eines oder mehrerer Oxide, Hydroxide oder wasserhaltiger Oxide eines oder mehrerer Elemente aus der Gruppe bestehend aus Aluminium, Silizium, Zirkonium, Zinn, Magnesium, Zink, Cer und Phosphor beschichtet ist.

3.  Transparentes Titandioxid-Sol enthaltend bis zu 20 Gew.-% nanodisperses Titandioxid gemäß Anspruch 1 oder 2 in einem oder mehreren Lösungsmitteln aus der Gruppe bestehend aus Wasser und Alkoholen, die 1 bis 10 Kohlenstoffatome und eine oder mehrere Hydroxidgruppen aufweisen.

4.  Verfahren zur Herstellung von nanodispersem Titandioxid gemäß Anspruch 1 oder 2, dadurch gekennzeichnet, daß

    a) zu einer alkalisch reagierenden Flüssigkeit bei erhöhter Temperatur eine schwefelsaure Titanylsulfatlösung zugegeben wird, bis die so erhaltene Mischung sauer reagiert, also Schwefelsäure im Überschuß vorliegt, oder eine alkalisch reagierende Flüssigkeit und eine schwefelsaure Titanylsulfatlösung bei erhöhter Temperatur simultan unter guter Vermischung in einem Behälter so zusammengeführt werden, daß die erhaltene Mischung sauer reagiert, also Schwefelsäure im Überschuß vorliegt,

b) die unter a) erhaltene Mischung abgekühlt wird,

c) anschließend eine einbasige Säure zu der nach b) erhaltenen Mischung zugegeben wird, wobei die unter a) gebildeten Titandioxidnanopartikel ausflocken,

d) der unter c) gebildete Flockungsniederschlag abfiltriert und mit der gleichen einbasigen Säure wie unter c) gewaschen wird.

5. Verfahren gemäß Anspruch 4, dadurch gekennzeichnet, daß die schwefelsaure Titanylsulfatlösung durch Auflösen von Titandioxid, Titandioxid-Hydraten, Titanaten oder Titanhalogeniden in Schwefelsäure erhalten wird und daß diese schwefelsaure Titanylsulfatlösung 100 bis 260 g/l Titan, berechnet als $TiO_2$, und zusätzlich zu dem als Titanylsulfat gebundenen Anteil 0,3 bis 4 mol Schwefelsäure pro mol $TiOSO_4$ enthält.

6. Verfahren gemäß Anspruch 4, dadurch gekennzeichnet, daß als alkalisch reagierende Flüssigkeit eine wäßrige Lösung von Natriumhydroxid, Kaliumhydroxid oder Ammoniak eingesetzt wird.

7. Verfahren gemäß Anspruch 4, dadurch gekennzeichnet, daß als schwefelsaure Titanylsulfatlösung eine sogenannte Schwarzlösung aus dem Titandioxid-Herstellungsprozeß nach dem Sulfatverfahren und als alkalisch reagierende Flüssigkeit Natronlauge eingesetzt wird.

8. Verfahren gemäß Anspruch 4, dadurch gekennzeichnet, daß der Reaktionsschritt a) bei Temperaturen zwischen 60 und 100°C durchgeführt wird.

9. Verfahren gemäß Anspruch 4, dadurch gekennzeichnet, daß nach dem Reaktionsschritt a) der pH-Wert der erhaltenen Mischung kleiner als 2 ist.

10. Verfahren gemäß Anspruch 4, dadurch gekennzeichnet, daß die in a) oder b) erhaltene Mischung durch Sedimentation, Filtration oder Zentrifugieren geklärt wird, bevor sie dem Reaktionsschritt b) oder c) unterzogen wird.

11. Verfahren gemäß Anspruch 4, dadurch gekennzeichnet, daß die einbasige Säure Salzsäure ist.

12. Verfahren zur Herstellung von transparentem Titandioxid-Sol gemäß Anspruch 3, dadurch gekennzeichnet, daß das Verfahren gemäß den Ansprüchen 4 bis 11 durchgeführt wird und anschließend der in d) angefallene Niederschlag in Wasser oder Alkoholen, die 1 bis 10 Kohlenstoffatome und eine oder mehrere Hydroxidgruppen aufweisen, aufgenommen wird, so daß sich ein transparentes Titandioxid-Sol bildet.

13. Verwendung des nanodispersen Titandioxids gemäß Anspruch 1 oder 2 als UV-Schutzkomponente in Kosmetika, Kunststoffen, Silikonharzen und Lacken.

14. Verwendung des nanodispersen Titandioxids gemäß Anspruch 1 oder 2 für den UV-katalysierten Abbau von Polymeren und organischen Schadstoffen in Abwässern.

15. Verwendung des nanodispersen Titandioxids gemäß Anspruch 1 oder 2 als Titandioxidkomponente in Farbstoff-Solarzellen.

## Claims

1. Nanodisperse titanium dioxide having a maximum value of the particle size distribution determined by means of an ultracentrifuge of between 1 and 10 nm, containing less than 0.1 wt.% of carbon in the form of organic compounds or residues and having a transparency of at least 99% measured in a 5 wt.% aqueous/hydrochloric acid solution between 400 and 700 nm in 180°/d geometry at a layer thickness of 10 μm.

2. Nanodisperse titanium dioxide according to claim 1, characterised in that the titanium dioxide is coated with 0.1 to 50 wt.%, relative to $TiO_2$, of one or more oxides, hydroxides or hydrous oxides of one or more elements from the group consisting of aluminium, silicon, zirconium, tin, magnesium, zinc, cerium and phosphorus.

3. Transparent titanium dioxide sol containing up to 20 wt.% of nanodisperse titanium dioxide according to claim 1 or 2 in one or more solvents from the group consisting of water and alcohols which contain 1 to 10 carbon atoms and one or more hydroxide groups.

4. Process for the production of nanodisperse titanium dioxide according to claim 1 or 2, characterised in that

   a) sulfuric-acid titanyl sulfate solution is added at elevated temperature to an alkaline-reacting liquid until the resultant mixture reacts acidically, *i.e.* sulfuric acid is present in excess, or an alkaline-reacting liquid and a sulfuric-acid titanyl sulfate solution are simultaneously brought together in a vessel at elevated temperature with thorough mixing in such a manner that the resultant mixture reacts acidically, *i.e.* sulfuric acid is present in excess,

   b) the mixture obtained in a) is cooled,

   c) a monobasic acid is then added to the mixture obtained in b), wherein the titanium dioxide nanoparticles formed in a) flocculate,

   d) the flocculate formed in c) is filtered out and washed with the same monobasic acid as in c).

5. Process according to claim 4, characterised in that the sulfuric-acid titanyl sulfate solution is obtained by dissolving titanium dioxide, titanium dioxide hydrates, titanates or titanium halides in sulfuric acid and that this sulfuric-acid titanyl sulfate solution contains 100 to 260 g/l of titanium, calculated as $TiO_2$ and, in addition to the proportion bound as titanyl sulfate, 0.3 to 4 mol of sulfuric acid per mol of $TiOSO_4$.

6. Process according to claim 4, characterised in that an aqueous solution of sodium hydroxide, potassium hydroxide or ammonia is used as the alkaline-reacting liquid.

7. Process according to claim 4, characterised in that a so-called black liquor from the titanium dioxide production process using the sulfate process is used as the sulfuric-acid titanyl sulfate solution and sodium hydroxide solution is used as the alkaline-reacting liquid.

8. Process according to claim 4, characterised in that reaction stage a) is performed at temperatures of between 60 and 100°C.

9. Process according to claim 4, characterised in that, after reaction stage a), the pH value of the resultant mixture is less than 2.

10. Process according to claim 4, characterised in that the mixture obtained in a) or b) is clarified by settling, filtration or centrifugation, before it is subjected to reaction stage b) or c).

11. Process according to claim 4, characterised in that the monobasic acid is hydrochloric acid.

12. Process for the production of a transparent titanium dioxide sol according to claim 3, characterised in that the process according to claims 4 to 11 is performed and the precipitate formed in d) is then resuspended in water or alcohols, which contain 1 to 10 carbon atoms and one or more hydroxide groups, such that a transparent titanium dioxide sol is formed.

13. Use of the nanodisperse titanium dioxide according to claim 1 or 2 as a UV screening component in cosmetics, plastics, silicone resins and lacquers.

14. Use of the nanodisperse titanium dioxide according to claim 1 or 2 for the UV-catalysed degradation of polymers and organic pollutants in waste waters.

15. Use of the nanodisperse titanium dioxide according to claim 1 or 2 as the titanium dioxide component in dye solar cells.

**Revendications**

1. Dioxyde de titane nanodispersé à un maximum de la répartition des dimensions de particules, mesuré à l'ultracentrifugeuse, de 1 à 10 nm, contenant moins de 0,1 % en poids de carbone sous la forme de composés organiques ou de résidus et présentant une transparence d'au moins 99 %, mesurée sur une solution aqueuse chlorhydrique à 5 % en poids entre 400 et 700 nm, à la géométrie de 180°/d et sur une épaisseur de couche de 10 µm.

2. Dioxyde de titane nanodispersé selon la revendication 1, caractérisé en ce qu'il est revêtu de 0,1 à 50 % de son poids d'un ou plusieurs oxydes, hydroxydes ou oxydes hydratés d'un ou plusieurs éléments du groupe consistant en l'aluminium, le silicium, le zirconium, l'étain, le magnésium, le zinc, le cérium et le phosphore.

3. Sol de dioxyde de titane transparent contenant jusqu'à 20 % en poids de dioxyde de titane nanodispersé selon la revendication 1 ou 2, dans un ou plusieurs solvants du groupe consistant en l'eau et les alcools en $C_1$-$C_{10}$ contenant un ou plusieurs groupes hydroxy.

4. Procédé de préparation du dioxyde de titane nanodispersé selon la revendication 1 ou 2, caractérisé en ce que

   a) à un liquide à réaction alcaline, on ajoute à haute température une solution sulfurique de sulfate de titanyle jusqu'à réaction acide du mélange obtenu, c'est-à-dire qu'on utilise l'acide sulfurique en excès, ou bien on coule un liquide à réaction alcaline et une solution sulfurique de sulfate de titanyle, simultanément sous bon mélange et à haute température dans un récipient, en sorte que le mélange obtenu présente une réaction acide, c'est-à-dire qu'il y a de l'acide sulfurique en excès,
   b) on refroidit le mélange obtenu en a) ci-dessus,
   c) au mélange obtenu en b), on ajoute ensuite un acide monobasique, ce qui provoque la floculation des nanoparticules de dioxyde de titane formées en a),
   d) on filtre le précipité de floculation formé en c) et on le lave avec le même acide monobasique qui a servi en c).

5. Procédé selon la revendication 4, caractérisé en ce que la solution sulfurique de sulfate de titanyle a été obtenue par dissolution de dioxyde de titane, de dioxyde de titane hydraté, de titanates ou d'halogénures du titane dans l'acide sulfurique et en ce que cette solution sulfurique de sulfate de titanyle contient 100 à 260 g/l de titane, exprimé en $TiO_2$, et, en plus de l'acide sulfurique fixé à l'état de sulfate de titanyle, 0,3 à 4 mol d'acide sulfurique par mole de $TiOSO_4$.

6. Procédé selon la revendication 4, caractérisé en ce que l'on utilise en tant que liquide à réaction alcaline une solution aqueuse d'hydroxyde de sodium, d'hydroxyde de potassium ou d'ammoniac.

7. Procédé selon la revendication 4, caractérisé en ce que l'on utilise en tant que solution sulfurique de sulfate de titanyle une "liqueur noire" de la préparation du dioxyde de titane par le procédé au sulfate, et en tant que liquide à réaction alcaline de la lessive de soude.

8. Procédé selon la revendication 4, caractérisé en ce que la température au stade de réaction a) est de 60 à 100°C.

9. Procédé selon la revendication 4, caractérisé en ce que, après le stade de réaction a), le pH du mélange obtenu est inférieur à 2.

10. Procédé selon la revendication 4, caractérisé en ce que le mélange obtenu en a) ou b) est clarifié par sédimentation, filtration ou centrifugation avant d'être soumis au stade de réaction b) ou c).

11. Procédé selon la revendication 4, caractérisé en ce que l'acide monobasique est l'acide chlorhydrique.

12. Procédé de préparation d'un sol transparent de dioxyde de titane selon la revendication 3, caractérisé en ce que l'on met en oeuvre le procédé selon les revendications 4 à 11, après quoi on reprend le précipité obtenu en d) dans de l'eau ou des alcools en $C_1$-$C_{10}$ contenant un ou plusieurs groupes hydroxy, ce qui donne le sol transparent de dioxyde de titane.

13. Utilisation du dioxyde de titane nanodispersé selon la revendication 1 ou 2, en tant que protecteur contre les UV dans des produits cosmétiques, des résines synthétiques, des résines de silicone et des vernis.

14. Utilisation du dioxyde de titane nanodispersé selon la revendication 1 ou 2 pour la dégradation, catalysée par les UV, de polymères et de substances organiques nocives dans des eaux résiduaires.

15. Utilisation du dioxyde de titane nanodispersé selon la revendication 1 ou 2 en tant que composant dioxyde de titane dans des cellules solaires à colorant.

Fig.1

# Fig. 2

Differentielle und integrale Teilchengrößenverteilung